# EUROPEAN PATENT APPLICATION

(11) **EP 3 409 787 A1**
(43) Date of publication of application: **05.12.2018**
(21) Application number: 17174010.3
(22) Date of filing: 01.06.2017
(51) Int. Cl.: C12Q 1/68

(54) **TWO SNPS (SINGLE NUCLEOTIDE POLYMORPHISMS) IN THE SEQUENCE OF THE BETA, BETA-CAROTENE 15,15 DIOXYGENASE GENE AND THEIR USE**

(71) Applicant: DSM IP Assets B.V., 6411 TE Heerlen (NL)
(72) Inventor: The designation of the inventor has not yet been filed
(74) Representative: Schwander, Kuno

(57) **Abstract**

The present invention relates generally to the field of diagnosis of Vitamin A deficiency. More particularly, it provides a method of diagnosing or detecting a predisposition or propensity or susceptibility for Vitamin A deficiency and Vitamin A deficiency disease (VADD). Specifically, the invention is directed to two single nucleotide polymorphism (SNP) markers, combinations of such markers associated with the risk of Vitamin A deficiency and to a method that comprises the steps of providing a biological sample of the subject to be tested and detecting the presence or absence of one or several genomic single nucleotide polymorphism (SNP) markers in the biological sample. Furthermore, the invention utilises both genetic and phenotypic information as well as information obtained by questionnaires to construct a score that provides the probability of Vitamin A deficiency and the combined preventive intervention by a dietary advice. In addition, the invention provides a kit to perform the method. The kit can be used to set a diagnosis of Vitamin A deficiency and of VADD for targeting of preventive interventions, such as said dietary advice.

## Description

### Field of the invention

The present invention relates generally to the field of diagnosis of Vitamin A deficiency. More particularly, it provides a method of diagnosing or detecting a predisposition or propensity or susceptibility for Vitamin A deficiency and Vitamin A deficiency disease (VADD).

Specifically, the invention is directed to two single nucleotide polymorphism (SNP) markers, combinations of such markers associated with the risk of Vitamin A deficiency and to a method that comprises the steps of providing a biological sample of the subject to be tested and detecting the presence or absence of one or several genomic single nucleotide polymorphism (SNP) markers in the biological sample. Furthermore, the invention utilises both genetic and phenotypic information as well as information obtained by questionnaires to construct a score that provides the probability of Vitamin A deficiency and the combined preventive intervention by a dietary advice. In addition, the invention provides a kit to perform the method. The kit can be used to set a diagnosis of Vitamin A deficiency and of VADD for targeting of preventive interventions, such as said dietary advice.

### Background of the invention

Vitamin A is essential for animals and humans. Vitamin A is formed exclusively in the animals and humans by cleavage of provitamin A carotenoids.

Vitamin A deficiency is associated with impaired vision, including night blindness and in severe cases xerophthalmia. In addition, vitamin A deficiency leads to impaired immune functions and an impaired barrier function of skin and mucosa, i.e. intestine.

Vitamin A deficiency can be prevented or reversed by the intake of a vitamin A supplement. This, however, is not without any risk: hypervitaminosis A can be toxic and may cause anorexia, dry itchy skin, alopecia, bone pain, increased intracranial pressure, and hepatosplenomegaly. During the first months of pregnancy, vitamin A supplementation in high doses is not recommended.

Beta-carotene is a well-known provitamin A source. Therefore, consumption of fruits and vegetables which are rich in beta-carotene can prevent vitamin A deficiency. However, the conversion of beta-carotene to vitamin A varies from person to person. Persons with a low conversion rate are referred to as low-responders, or more correctly as low-converters. Maintaining a healthy vitamin A level is particularly critical if a low-responder is a vegetarian, not consuming food rich in vitamin A such as beef liver or cod liver oil.

Vitamin A deficiency disease (VADD) affects millions of people, especially in Asia and Africa. 250 to 300 millions of pre∼school-age children are subclinically vitamin A deficient. 3 millions have a severe clinical vitamin A deficiency and 500'000 children go blind every year due to xerophtalmia. As VADD also strongly affects the immune response, the majority of them die within one year. The population in most Asian and African countries relies on rice and grains as staple food. They do not consume a lot of carotenoid rich fruits and vegatables. In addition, a significant number of people have an impaired absorption and cleavage of provitamin A carotenoids.

Therefore these people are at risk for subclinical if not clinical VADD and should be identified early in life.

Chronic intake of foods low in vitamin A (retinol) and provitamin A forming an unbalanced diet with little variety is common in young individuals and can lead to subclinical micronutrient deficiency. Provitamin A sources such as β -carotene are cleaved centrally by the β, β -carotene 15,15' dioxygenase (BCO1) into retinal, the precursor of retinol. However, the amount of β - carotene and retinol produced after ingestion of β -carotene is highly variable between healthy individuals, with approximately 40% of the subjects being classified as low responders. Several stable isotope studies have shown a large disparity between the most efficient converters and the most inefficient converters of β-carotene with variations of up to 8 fold. It is possible that differences in β-carotene response may be due to genetic polymorphisms in genes involved in aspects of β-carotene conversion or uptake.

### Summary of the invention

It was found in accordance with the present invention that two SNP markers exist that are associated with poor β-carotene cleavage activity resulting in the risk of developing a vitamin A deficiency.

Thus the present invention provides two individual SNP markers associated with the risk of developing a vitamin A deficiency and with VADD. In particular, the present invention comprises two SNPs (single nucleotide polymorphisms) in the coding region of the β, β -carotene 15,15'-dioxygenase gene (SNP1 rs12934922 and SNP2 rs7501331). Functional testing of a protein bearing the mutation at amino acid position No. 267 (SNP1: Arginin -> Serin / R267S) gave rise to a protein with altered β, β -carotene 15,15'-monooxygenase activity. The same observation was made for an amino acid change at position No. 379 (SNP2 Alanine -> Valin / A379V). The two polymorphisms can serve as an early marker for vitamin A deficiency.

In Case of SNP2, rs7501331 SNP results in an exchange of an amino acid in the β, β -carotene 15,15'-dioxygenase polypeptide at position 379, wherein alanine is replaced by valine. For this replacement, the notation "Ala379Val" or "A379V: rs7501331" is used. Thus, CC carriers (wildtype) of rs7501331 have alanine at position 379 of beta-carotene 15,15'-monooxygenase whereas TT carriers of rs7501331 have valine at position 379 of beta-carotene 15,15'-monooxygenase.

A SNP resulting in an exchange of an amino acid in the corresponding enzyme often has a certain effect. In the present case, CC carriers of rs7501331 have a lower Total Body Stores (TBS) of vitamin A than CT and TT carriers of rs7501331. This is surprising if one considers that approximately 56% of the human population are CC carriers of rs7501331. In other words, having A379V: rs7501331 polymorphism is beneficial in terms of the Total Body Stores (TBS) of vitamin A, compared to the wild type. This is very surprising because SNPs are rarely beneficial for an individual and often underlie differences in one's susceptibility to disease.

The person skilled in art knows how to determine the Total Body Stores (TBS) of vitamin A, e.g. by determining the [¹³C₁₀] retinyl acetate dose after a sufficient period (circa 3 days) of isotope dilution with endogenous pools. This is further described in Oxley, A. et al, 2014, An LC/MS/MS method for stable isotope dilution studies of β-carotene bioavailability, bioconversion, and vitamin A status in humans, J- Lipid Res. 55, 319-328. In well-nourished individuals, the liver contains ≥90% of the Total Body Stores of vitamin A.

Wildtype CC carriers of rs7501331 are more likely to suffer from vitamin A deficiency or insufficiency because they have a relatively low Total Body Stores of vitamin A. Typically, a blood-serum retinol concentration of 0.70 µmol/l or less refers to "vitamin A deficiency" whereas blood-serum retinol concentrations between 0.70 µmol/l and 1.40 µmol/l refer to "vitamin A insufficiency". In the context of the present invention, the expression "vitamin A deficiency" refers preferably to both, "vitamin A deficiency" and "vitamin A insufficiency". In an alternative embodiment of the invention, the expression "vitamin A deficiency" refers "vitamin A deficiency" only, meaning a blood-serum retinol concentration of 0.70 µmol/l or less.

Providing a vitamin A supplement to everybody in order to avoid Vitamin A deficiency or VADD is not recommended because such an approach may result in unwanted side effects or even toxic hypervitaminosis. Furthermore, vitamin A supplements are not storage stable and therefore difficult to commercialize.

Surprisingly, a daily intake of for example 7 mg beta-carotene can overcome the single nucleotide polymorphism (A379V: rs7501331) effect in the beta-carotene converting enzyme beta-carotene 15,15'-monooxygenase, particularly if said 7 mg beta-carotene are formulated in suitable manner. In other words, CC carriers of rs7501331 benefit more from beta-carotene supplementation than TT carriers of the rs7501331. This is shown in a clinical study (Example 1) underlying the present invention. Such an approach is promising because hypervitaminosis A due to a higher than normal intake of beta-carotene has never been observed so far. Due to a feedback mechanism, the biologically active vitamin A metabolite retinoic acid is regulating the uptake and the cleavage of beta-carotene via the intestinal factor ISX. As an additional benefit, compositions comprising beta-carotene are most often more storage stable than vitamin A.

Similar effect is linked to SNP1 wherein the amino acid change at position No. 267 also results in an alteration of β, β -carotene 15,15'-monooxygenase activity.

Beta-carotene supplements are commercially available as powders, capsules, beverages and other formulations. The crucial role of the beta-carotene formulation as a determinant of the bioavailability of beta-carotene has long been recognized. Thürmann et al. studied the plasma concentration response to drinks containing beta-carotene formulated as a water dispersible powder (cf. Thürmann P.A, Steffen J, Zwernemann C, Aebischer C-P, Cohn W, Schalch W., Plasma concentration response to drinks containing β-carotene as carrot juice or formulated as a water dispersible powder, Eur J Nutr 2002; 41:228-235). As water dispersible powder, Thürmann et al. used "carotene 10% CWS", a product comprising 4 weight-% of oil.

The polymorphisms according to the present invention can be used in nutrition and diagnostics in order to identify parts of the population with poor β -Carotene cleavage activity, who need further Vitamin A supplementation. Furthermore it can be used in African and Asian countries in order to identify people, especially children with a subclinical Vitamin A deficiency. It can be further used to assist people with a higher Vitamin A need, i.e. pregnant and lactating women and to set personalized nutritional recommendations.

Therefore, the invention relates to a method for estimating susceptibility or predisposition of an individual to VADD comprising the detection of the presence/un-presence of the SNP markers and haplotypes. In particular, the present invention is related to the use of the SNP markers defined above for selecting a diet comprising beta-carotene either alone or as a major component an individual should be provided in order to prevent or treat Vitamin A deficiency and to a method of identifying the risk of developing a Vitamin A deficiency and/or VADD by detecting SNP markers in a biological sample of the subject. The information obtained from this method can be combined with other information concerning an individual, e.g. results from blood measurements, clinical examination and questionnaires. The information to be collected by questionnaire includes information concerning gender, age, family and medical history such as the family history.

The invention further relates to a kit for estimating susceptibility to Vitamin A deficiency or VADD in an individual comprising wholly or in part: amplification reagents for amplifying nucleic acid fragments containing SNP markers, detection reagents for genotyping SNP markers and interpretation software for data analysis and risk assessment.

Those skilled in the art will readily recognize that the analysis of the nucleotides present in one or several of the SNP markers of this invention in an individual's nucleic acid can be done by any method or technique capable of determining nucleotides present in a polymorphic site. As it is obvious in the art the nucleotides present in SNP markers can be determined from either nucleic acid strand or from both strands.

### Detailed description of the invention

In summary, the present invention also relates to the following aspects:
1. The use of at least one single nucleotide polymorphism (SNP) in the β, β -carotene 15,15'-dioxygenase gene for selecting a diet an individual should be provided to prevent or treat Vitamin A deficiency or Vitamin A deficiency disease (VADD) during an intervention program.
2. The use of the single nucleotide polymorphisms according to claim 1, wherein the SNP is in the coding region of the β, β -carotene 15,15'-dioxygenase gene.
3. The use according to claim 1 or 2, wherein the single nucleotide polymorphism is rs12934922 and wherein the corresponding mutation is at position 1019 resulting in an amino acid change in amino acid position No. 267 replacing an arginin with a serin.
4. The use according to any of claims 1 to 4, wherein the single nucleotide polymorphism is rs7501331 and wherein the corresponding mutation is at position 1354 resulting in an amino acid change in amino acid position No. 379 replacing an alanine with a valin.
5. The use according to claim 4, wherein the un-presence of the mutation triggers the selection of a diet an individual should be provided to prevent or treat Vitamin A deficiency or Vitamin A deficiency disease (VADD).
6. The use according to any of claims 1 to 5, wherein the individual has A deficiency or Vitamin A deficiency disease.
7. The use according to any of claims 1 to 6, wherein the diet an individual should be provided is characterized by an additional beta-carotene supplementation.
8. A method for the identification of an individual who has an altered risk for developing Vitamin A deficiency or VADD, the method comprising the steps of
   a. providing a biological sample taken from a subject;
   b. determining the nucleotides present in at least one of the polymorphic sites as set forth in claims 3 to 5 in said individual's nucleic acid; and
   c. combining the SNP marker data to assess the risk of an individual to develop VADD.
9. The method according to claim 8, further comprising a step of combining the genetic information with personal and clinical information of said individual to assess the risk of an individual to develop VADD.
10. The method according to claim 9, wherein the personal and clinical information concerns age, gender, behaviour patterns and habits, biochemical measurements, clinical measurements, family history of VADD.
11. A test kit for determining the nucleotides present in at least one of the polymorphic sites as set forth in claims 3 to 5 in said individual's nucleic acid for assessment of an altered risk of VADD in a subject.

In an aspect, the invention relates to the use of at least one genetic marker, e.g. a SNP, for determining what type of diet an individual may be given in order to avoid vitamin A deficency. The marker determines the likelihood that an individual developes a vitamin A deficiency under a traditional or normal diet. An "individual" as used in the present application refers to a vertebrate, preferably a mammal, more preferably an animal such as a domestic animal (e.g. a dog or cat), and most preferably a human.

As used herein "polymorphism" refers to DNA sequence variation in the cellular genome of an individual, typically with a population frequency of more than 1%. A polymorphic marker or site is the locus at which genetic variation occurs. The two SNP markers have two alleles, each occurring at frequency of greater than 1%, and more preferably greater than 10% or 20% of a selected population. The first identified allelic form is arbitrarily designated as the reference form and other allelic forms are designated as alternative or variant alleles. The allelic form occurring most frequently in a selected population is sometimes referred to as the wild-type form. Diploid organisms may be homozygous or heterozygous for allelic forms. A SNP occurs at a polymorphic site occupied by a single nucleotide. A SNP usually arises due to substitution of one nucleotide for another at the polymorphic site, but it can also arise from an insertion or deletion of a nucleotide relative to a reference allele. The two SNPs according to the present invention are characterized by the substitution of one nucleotide at the polymorphic site resulting in three groups of carriers. In case of SNP2 (rs7501331) the three possible groups of carriers are: CC (wildtype, homozygous), CT (heterozygous) and TT (Polymorphismus, homozygous), wherein - as mentioned above - wildtype CC carriers of rs7501331 are more likely to suffer from vitamin A deficiency or insufficiency.

"Biological sample" as used in the present invention encompasses a variety of sample types that can be used as source material for isolating nucleic acids. They include, but are not limited to, solid materials (*e.g*., tissue, tissue cultures or cells derived there from and the progeny thereof, hair follicle samples, biopsy specimens, buccal cells provided by a swab, skin and nose samples) and biological fluids (*e.g*. urine, faecal material, blood, semen, amniotic fluid, tears, saliva, sputum, sweat, mouth wash). Any biological sample from a human individual comprising even one cell comprising nucleic acid can be used in the methods of the present invention. The term also includes samples that have been manipulated in any way after their procurement, such as by treatment with reagents, solubilisation, or enrichment for certain components, such as proteins or polynucleotides. The methods and uses of the present invention are preferably conducted on a sample that has previously been removed from the individual and do preferably not involve diagnosis practised on the human body.

Nucleic acid molecules as used herein refers to polymeric forms of nucleotides and includes both sense and antisense strands of RNA, cDNA, genomic DNA, and synthetic forms and mixed polymers of the above, with genomic DNA being preferred. A nucleotide refers to a ribonucleotide, deoxynucleotide or a modified form of either type of nucleotide. The term also includes single- and double-stranded forms of DNA. In addition, a polynucleotide may include either or both naturally-occurring and modified nucleotides linked together by naturally-occurring and/or non-naturally occurring nucleotide linkages. The nucleic acid molecules may be modified chemically or biochemically or may contain non-natural or derivatized nucleotide bases, as will be readily appreciated by those of skill in the art. Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. A reference to a nucleic acid sequence encompasses its complement unless otherwise specified. Thus, a reference to a nucleic acid molecule having a particular sequence should be understood to encompass its complementary strand, with its complementary sequence. The complementary strand is also useful, e.g., for antisense therapy, hybridization probes and PCR primers.

Nucleic acids can be isolated from a particular biological sample using any of a number of procedures, which are well-known in the art, the particular isolation procedure chosen being appropriate for the particular biological sample. Methods of isolating and analyzing nucleic acid variants as described above are well known to one skilled in the art and can be found, for example in the Molecular Cloning: A Laboratory Manual, 3rd Ed., Sambrook and Russel, Cold Spring Harbor Laboratory Press, 2001 and Current Protocols in Molecular Biology Volumes I-III, 4th edition, Ausubel et al., John Wiley and Sons, 1995. Many of the methods require amplification of nucleic acid from target samples. This can be accomplished by techniques such as e.g. PCR, ligase chain reaction, nucleic acid based sequence amplification, self-sustained sequence replication and transcription amplification. Genetic markers such as the SNPs can be detected from the isolated nucleic acids using techniques including DNA hybridization methods (e.g. Southern Blotting, FISH), direct sequencing with radioactively, enzymatically, luminescently or fluorescently labelled primers (manually or automated), restriction fragment length polymorphism (RFLP) analysis, heteroduplex analysis, single strand conformational polymorphism (SSCP) analysis, denaturing gradient gel electrophoresis (DGGE), temperature gradient gel electrophoresis (TGGE), use of linked genetic markers, mass spectrometry e.g. MALDI-TOF, and chemical cleavage analysis to name just a few. Of course DNA MicroArray technology suitable for detecting genetic markers such as SNPs can also be used. All methods are explained in detail, for example, in the Molecular Cloning: A Laboratory Manual, 3rd Ed., Sambrook and Russel, Cold Spring Harbor Laboratory Press, 2001.

Primers used may be oligonucleotides hybridizing specifically with one allele. They are called allele-specific oligonucleotides. In the allele-specific PCR methodology, a target DNA is preferentially amplified only if it is completely complementary to the 3'-terminus of a specific PCR amplification primer. The 3'-terminus of the primer is designed so as to terminate at, or within one or two nucleotides of a known mutation site within the target DNA to which it possesses a complementary sequence. Under the appropriate reaction conditions, the target DNA is not amplified if there is a single nucleotide mismatch (e.g., a nucleotide substitution caused by a mutation) or a small deletion or insertion, at the 3'-terminus of the primer. Accordingly, allele-specific PCR may be utilized to detect either the presence or absence of (at least) a single nucleotide mismatch between the primer sequence (which is complementary to a pre-selected target sequence) and a nucleic acid within the sample. Amplification of the target sequence is indicative of a lack of even a single mismatched nucleotide. The markers in the present invention are preferably analyzed using methods amenable for automation. Primer extension analysis can be performed using any method known to one skilled in the art. Oligonucleotides, probes and/or primers may be naturally occurring or synthetic, but are typically prepared by synthetic means. They may be immobilized on a solid support. For instance, oligonucleotides, probes and/or primers as described herein can be used as a DNA chip. The chip may contain a primer corresponding to a single allelic form of a marker but may also contain a primer corresponding to both allelic forms of a marker. It may even comprise primers for different markers. The appropriate length of an oligonucleotide, probe and/or primer depends on its intended use but typically ranges from 10 to 75, preferably 15 to 40 nucleotides. Short primer molecules generally require cooler temperatures to form sufficiently stable hybrid complexes with the template. A primer need not reflect the exact sequence of the template but must be sufficiently complementary to hybridize with a template. Conditions suitable for hybridization are generally known in the art and will be apparent to the skilled artisan. A nonlimiting example of stringent hybridization conditions is hybridization in 6 x sodium chloride/sodium citrate (SSC) at about 45°C, followed by one or more washes in 0.2 x SSC, 0.1% SDS at 50-65°C. Stringent conditions can for instance be found in Molecular Cloning: A Laboratory Manual, 3rd Ed., Sambrook and Russel, Cold Spring Harbor Laboratory Press, 2001 and Current Protocols in Molecular Biology, John Wiley & Sons, N.Y. (1989), 6.3.1-6.3.6. The term "primer site" refers to the area of the target DNA to which a primer hybridizes. The term "primer pair" means a set of primers including a 5'-upstream primer that hybridizes with the 5'-end of the DNA sequence to be amplified and a 3'-downstream primer that hybridizes with the complement of the 3'-end of the sequence to be amplified.

As used herein, "genotype" refers to the genetic constitution of an individual. More specifically, "genotyping" as used herein refers to the analysis of DNA in a sample obtained from a subject to determine the DNA sequence in a specific region of the genome, *e.g*. a locus that influences a trait. It may refer to the determination of DNA sequence at one or more polymorphic sites and/or determination of allelic patterns of an individual. The genotyping may be performed using a micro-array or a multi-well plate in for instance a laboratory or hospital. It may thus involve the use of a gene/DNA chip or a strip or solid surface comprising one or more nucleic acid molecules.

The steps of obtaining a sample from an individual, determining the genotype of a SNP, e.g. SNP1 rs12934922 and SNP2 rs7501331 in the β, β -carotene 15,15'-dioxygenase gene, and linking the genotype of the SNP to a preventive intervention by a dietary advice, that is more or less suitable for the individual. The steps can be done by one party, however, the steps can also be performed by two or even more distinct parties.

Another aspect of the invention is directed to a kit for use in a method or use of the present invention. The kit comprises at least one primer or primer pair for genotyping the SNP1 or SNP2 marker. The primers may be suitable for nucleic acid sequence amplification. Often the kits contain one or more primers or primer pairs hybridizing to different forms of a polymorphism, e.g. a primer or primer pair capable of hybridizing to the A allelic form of SNP1 rs12934922 and a primer or primer pair capable of hybridizing to the T allelic form of SNP1 rs12934922. Moreover, kits according to the invention may comprise instructions explaining correlation of the genotype to the predisposition or propensity or susceptibility for Vitamin A deficiency and VADD. In case the marker is SNP2, the instructions may explain that detection of two T allelic forms of the SNP is indicative of the individual as being more likely to suffer from a Vitamin A deficiency and VADD. Optional additional components of the kit include, for example, restriction enzymes, reverse transcriptase or polymerase, a positive control, a negative control, at least a further primer pair suitable for detecting (other) markers, appropriate buffers for reverse transcription, PCR and/or hybridization reactions, means used to label and nucleotide mix for the PCR reaction.

Polyclonal and/or monoclonal antibodies that specifically bind one form of the gene product but not to the other form of the gene product are also provided. In general, antibodies can be used diagnostically to monitor protein levels in tissue such as blood as part of a test predicting the susceptibility to VADD or as part of a clinical testing procedure, e.g., to, for example, determine the efficacy of a given treatment regimen. Detection can be facilitated by coupling the antibody to a detectable substance, which according to the invention is the β, β -carotene 15,15' dioxygenase (BCO1).

Antibodies are also provided that bind a portion of either the variant or the reference gene product that contains the polymorphic site or sites. The term "antibody" as used herein refers to immunoglobulin molecules and immunologically active portions of immunoglobulin molecules, i.e., molecules that contain an antigen binding site that specifically binds an antigen. A molecule that specifically binds to a polypeptide of the invention is a molecule that binds to that polypeptide or a fragment thereof, but does not substantially bind other molecules in a sample, e.g., a biological sample, which naturally contains the polypeptide. Examples of immunologically active portions of immunoglobulin molecules include F(ab) and F(ab') fragments which can be generated by treating the antibody with an enzyme such as pepsin. The invention provides polyclonal and monoclonal antibodies that bind to a polypeptide of the invention. The term "monoclonal antibody" or "monoclonal antibody composition", as used herein, refers to a population of antibody molecules that contain only one species of an antigen binding site capable of immunoreacting with a particular epitope of a polypeptide of the invention. A monoclonal antibody composition thus typically displays a single binding affinity for a particular polypeptide of the invention with which it immunoreacts.

Polyclonal antibodies can be prepared as known by those skilled in the art by immunizing a suitable subject with a desired immunogen, e.g., polypeptide of the invention or fragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (e.g., from the blood) and further purified by well-known techniques, such as protein A chromatography to obtain the IgG fraction. At an appropriate time after immunization, e.g., when the antibody titers are highest, antibody-producing cells can be obtained from the subject and used to prepare monoclonal antibodies by standard techniques, such as the hybridoma technique (Kohler G and Milstein C, 1975), the human B cell hybridoma technique (Kozbor D et al, 1982), the EBV-hybridoma technique (Cole SP et al, 1994), or trioma techniques (Hering S et al, 1988). To produce a hybridoma an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide of the invention.

Any of the many well known protocols used for fusing lymphocytes and immortalized cell lines can be applied for the purpose of generating a monoclonal antibody to a polypeptide of the invention (Bierer B et al, 2002). Moreover, the ordinarily skilled worker will appreciate that there are many variations of such methods that also would be useful.

Alternative to preparing monoclonal antibody-secreting hybridomas, a monoclonal antibody to a polypeptide of the invention can be identified and isolated by screening a recombinant combinatorial immunoglobulin library (e.g., an antibody phage display library) with the polypeptide to thereby isolate immunoglobulin library members that bind the polypeptide (Hayashi N et al, 1995; Hay BN et al, 1992; Huse WD et al, 1989; Griffiths AD et al, 1993). Kits for generating and screening phage display libraries are commercially available.

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art.

In one embodiment of the invention, diagnosis of VADD or susceptibility to VADD (or diagnosis of or susceptibility to a disease or condition associated with VADD), is made by detecting one or several of polymorphic sites which are associated with at-risk alleles or/and at-risk haplotypes described in this invention in the subject's nucleic acid. Diagnostically the most useful polymorphic sites are those altering the polypeptide structure of a VADD associated gene due to an amino acid change. Nucleotide changes in SNP1 and SNP2 resulting in a change in polypeptide sequence in alter the physiological properties/activity of the β, β -carotene 15,15' dioxygenase (BCO1).

In diagnostic assays determination of the nucleotides present in one of the VADD associated SNP markers of this invention, as well as polymorphic sites associated with VADD associated SNP markers of this invention, in an individual's nucleic acid can be done by any method or technique which can accurately determine nucleotides present in a polymorphic site. Numerous suitable methods have been described in the art (Kwok P-Y, 2001; Syvänen A-C, 2001), these methods include, but are not limited to, hybridization assays, ligation assays, primer extension assays, enzymatic cleavage assays, chemical cleavage assays and any combinations of these assays. The assays may or may not include PCR, solid phase step, modified oligonucleotides, labeled probes or labeled nucleotides and the assay may be multiplex or singleplex. As it is obvious in the art the nucleotides present in polymorphic site can be determined from one nucleic acid strand or from both strands.

In another embodiment of the invention, diagnosis of a susceptibility to VADD can also be made by examining transcription of at least one VADD associated gene, preferably of the β, β - carotene 15,15' dioxygenase gene. Alterations in transcription can be analysed by a variety of methods as described in the art, including e.g. hybridization methods, enzymatic cleavage assays, RT-PCR assays and microarrays. A test sample from an individual is collected and the alterations in the transcription of the VADD associated gene is assessed from the RNA present in the sample. Altered transcription is diagnostic for a susceptibility to VADD.

In another embodiment of the invention, diagnosis of a susceptibility to VADD can also be made by examining expression and/or structure and/or enzymatic activity of β, β -carotene 15,15' dioxygenase (BCO1). A test sample from an individual is assessed for the presence of an alteration in the expression and/or an alteration in structure and/or enzymatic activity of the polypeptide encoded by the VADD risk gene, or for the presence of a particular polypeptide variant (e.g., an isoform) encoded by the VADD risk gene. An alteration in expression of the polypeptide encoded by the VADD risk gene can be, for example, an alteration in the quantitative polypeptide expression.

Alterations in expression and/or structure and/or enzymatic activity of the VADD susceptibility polypeptide (the β, β -carotene 15,15' dioxygenase1) can be determined by various methods known in the art e.g. by assays based on chromatography, spectroscopy, colorimetry, electrophoresis, isoelectric focusing, specific cleavage, immunologic techniques and measurement of biological activity as well as combinations of different assays. An "alteration" in the polypeptide expression or composition, as used herein, refers to an alteration in expression or composition in a test sample, as compared with the expression or composition of polypeptide by the VADD risk gene in a control sample. A control sample is a sample that corresponds to the test sample (e.g., is from the same type of cells), and is from an individual who is not affected by VADD. An alteration in the expression or composition of the polypeptide in the test sample, as compared with the control sample, is indicative of a susceptibility to VADD.

Western blotting analysis, using an antibody as described above that specifically binds to a polypeptide encoded by the mutant VADD risk gene, or an antibody that specifically binds to a polypeptide encoded by a non-mutant gene, or an antibody that specifically binds to a particular splicing variant encoded by the VADD risk gene, can be used to identify the presence in a test sample of a particular splicing variant or isoform, or of a polypeptide encoded by the polymorphic or mutant VADD risk gene, or the absence in a test sample of a particular splicing variant or isoform, or of a polypeptide encoded by tha non-polymorphic or non-mutant gene. The presence of a polypeptide encoded by the polymorphic or mutant gene, or the absence of a polypeptide encoded by the non-polymorphic or non-mutant gene, is diagnostic for a susceptibility to VADD.

In one embodiment of this method, the level or amount of polypeptide encoded by the VADD risk gene in a test sample is compared with the level or amount of the polypeptide encoded by the VADD risk gene in a control sample. A level or amount of the polypeptide in the test sample that is higher or lower than the level or amount of the polypeptide in the control sample, such that the difference is statistically significant, is indicative of an alteration in the expression of the polypeptide encoded by the VADD risk gene, and is diagnostic for a susceptibility to VADD.

Kits (e.g., reagent kits) useful in the methods of diagnosis comprise components useful in any of the methods described herein, including for example, PCR primers, hybridization probes or primers as described herein (e.g., labeled probes or primers), reagents for genotyping one or several polymorphic markers, reagents for detection of labeled molecules, restriction enzymes (e.g., for RFLP analysis), allele-specific oligonucleotides, DNA polymerases, RNA polymerases, marker enzymes, antibodies which bind to altered or to non-altered (native) VADD susceptibility polypeptide, means for amplification of nucleic acids comprising the VADD risk gene (the β, β - carotene 15,15' dioxygenase gene), or means for analyzing the nucleic acid sequence of the VADD risk gene or for analyzing the amino acid sequence of β, β -carotene 15,15' dioxygenase.

In one embodiment, a kit for diagnosing susceptibility to VADD can comprise primers for nucleic acid amplification of a region in the VADD risk gene comprising an at-risk haplotype that is more frequently present in an individual susceptible to VADD. The primers can be designed using portions of the nucleic acids flanking SNPs that are indicative of VADD.

This invention is based on the principle that one or a small number of genotypings are performed, and the mutations to be typed are selected on the basis of their ability to predict VADD. For this reason any method to genotype mutations in a genomic DNA sample can be used. If non-parallel methods such as real-time PCR are used, the typings are done in a row. The PCR reactions may be multiplexed or carried out separately in a row or in parallel aliquots.

Thus, the diagnostic assay of the invention may further comprise a step of combining information concerning age, gender, the family history of VADD, with the results obtained from the analytical step described above.

The diagnostic assay or test can be applied to test the risk of developing VADD in both healthy persons, as a screening or predisposition test, and in high-risk persons (who have e.g. family history of VADD).

The method can be used in the prediction and early diagnosis of VADD in adult persons, stratification and selection of subjects in clinical trials, stratification and selection of persons for intensified preventive and curative interventions. The aim is to reduce the cost of clinical drug trials and health care.

### EXAMPLES

To illustrate the invention, the following examples are provided. These examples are not intended to limit the scope of the invention.

### Example 1: Study: Effect of SNPs in the β-carotene 15,15'-monooxygenase enzyme on retinol formation and β-carotene plasma responses

The study underlying the present invention analyzed *inter alia* the Total Body Store of vitamin A (TBS) using an isotope dilution method and [¹³C₁₀] retinol concentrations, and determined the effect of the rs7501331 or rs6564851 SNPs on BCO1 enzyme activity. To enable the analysis of SNPs on TBS, stepwise multiple regression was performed using the Akaike Information Criterion (AIC) to evaluate the covariates dietary intake of retinol, provitamin A carotenoids and retinol equivalents, age, BMI and body fat. The most significant covariant used for the selective model to analyze the SNP and TBS interaction was dietary retinol equivalent, with an AIC of 276.5. Thus, the final model tested the effects of the rs7501331 or rs6564851 SNPs on TBS at both baseline and after 54 days of supplementation accounting for dietary retinol equivalent as the only co-variant in the analysis. Further details are given in the following sections.

7 mg beta-carotene supplements were given daily over a period of 54 days to human subjects via oral capsules containing three different formulations:
Formulation A (BetaTab 20% S), administered to group A
Formulation B (β-carotene 15% LCS), administered to group B
Formulation C (β-carotene 30% FS), administered to group C

### Study design:

The study was a randomized, single blind supplementation design combined with retrospective genotyping to test three beta-carotene formulations (7mg β-carotene/day) for a period of 8 weeks. The three different β-carotene formulations were as follows: BetaTab 20% S; β-carotene 15% LCS; β-carotene 30% FS. On day 1 and 57 of each supplementation period, a fasting blood sample was taken before the oral administration of 2 mg of [¹³C₁₀] β-carotene and 0.4 mg of [¹³C₁₀] retinyl acetate as part of a test breakfast meal. Further blood samples were taken at 2, 4, 6, 8, 10 and 12 hours post-dose. For the first formulation (BetaTab 20%S) single fasting blood samples were also taken at day 2, 3, 4, 8, 10, 22, 36 and day 57 of the 8 week supplementation period followed by fasting blood samples at day 58, 59, 60, 64, 66, 78, 92 and 113 during the wash-out period. For formulations 2 and 3 (β-carotene 30% FS; β-carotene 15% LCS) additional fasting blood samples were taken at day 4 and day 60. The total duration period for supplement 1 is 16 weeks due to the additional blood collection necessary for the modelling of retinol body stores and plasma flux. The study duration for supplements 2 and 3 was 9 weeks. Additional stable isotope measurements at day 4 and day 60 during supplements 2 and 3 were sufficient to estimate retinol liver stores, but did not give sufficient information on retinol flux. However, overall body retinol homeostasis was determined using supplement 1 (BetaTab 20% S).

### Material:

The three beta-carotene formulations were supplied by DSM and are commercially available at DSM. Differences in the qualitative and quantitative composition of the three formulations and a summary of the study result is given below:

| | **BetaTab 20% S** | **β-carotene 15% LCS** | **β-carotene 30% FS** |
|---|---|---|---|
| **appearance** | free-flowing particles | free-flowing particles | free-flowing particles |
| **dispersibility in water** | dispersible | dispersible | not dispersible in water |
| **color of aqueous dispersion** | red/orange | no to very low color saturation | n.a. |
| **size of particles dispersed in water** (Malvern, D[4,3], Fraunhofer) | less than 2 µm | from 5.0 µm to 9 µm | n.a. |
| **beta-carotene content** | 20 weight-% | 15 weight-% | 30 weight-% |
| **oil content** | 1.5 weight-% | 0 weight-% | 69.3 weight-% |
| **modified food starch** | 50 weight-% | 57 weight-% | 0 weight-% |
| **corn starch** | 20.5 weight-% | 0 weight-% | 0 weight-% |
| **maltodextrin** | 0 weight-% | 25.8 weight-% | 0 weight-% |
| **other excipients** | 8 weight-% | 2.2 weight-% | 0.7 weight-% |
| **comparison of formulations A, B and C** | Time to reach plasma concentration maximum was on average around 40-47 days. Plasma β-carotene concentration increased depended on the formulation. It was highest in group A with 2.09 ± 1.00 µmol/L, whereas the difference between formulation B and C was not statistically significant. Similarly, Total Body Stores (TBS) was increased by the supplementation with all three formulations. The largest increases in TBS were observed in volunteers consuming supplement A (28.1 µM; 40.4%). | | |

### Study results:

The data show higher Total Body Store (TBS) concentrations in volunteers with the rs7501331 T variant at baseline. rs7501331 CT carriers had significantly higher TBS at baseline than CC carriers (p=0.041; cf. Table). Combining the CT and TT carriers of rs7501331 and comparing these to CC carriers revealed that CT/TT carriers had significantly higher TBS at baseline compared to CC carriers (Table 2, p=0.022). Importantly, coding the rs7501331 genotype as continuous covariate (i.e. 0=CC, 0.5=CT and 1=TT) did confirm the additive impact of the T allele on TBS (p=0.018), thus confirming that the T variant of rs7501331 had higher TBS concentrations than the C variant (cf. Table 1).

Surprisingly, any significant effect of the dominant T variant on TBS values disappeared after ß-carotene supplementation.

On the contrary, the data indicate that any effect of the rs6564851 SNPs does not disappear after ß-carotene supplementation. Compared to GG carriers of rs6564851, GT carriers had significantly higher TBS concentrations at the end of the supplementation.

### Summary:

A daily intake of BetaTab 20% S, β-carotene 15% LCS or β-carotene 30% FS in an amount corresponding to 7 mg beta-carotene per day can compensate the lower Total Body Store (TBS) concentrations in volunteers being CC carriers of rs7501331.

The formulation β-carotene 30% FS contains a significant amount of oil and thus, is not dispersible in water. The BetaTab 20% S is dispersible in water, but cannot be used to manufacture a colorless beverage. β -carotene 15% LCS is the preferred composition because it be used to manufacture a colorless, storage stable beverage.

**Table1 : Total Body Stores of vitamin A (TBS) at baseline and end of intervention depending on BCO1 SNPs combining dominant SNP variants**

| | | **rs7501331** | | **rs6564851** | |
|---|---|---|---|---|---|
| | | **CC** | **CT/TT** | **GG** | **GT/TT** |
| Day 4 TBS (µmol) | Mean | **64.1** | **79.9*** | **61.7** | **73.2** |
| | 95% CI | 57.2-71.9 | 68.8-92.7 | 52.1-72.9 | 65.6-81.7 |
| | | | | | |
| Day 60 TBS (µmol) | Mean | **76.8** | **78.4** | **67.1** | **82.4** |
| | 95% CI | 67.5-87.4 | 65.6-93.7 | 55.8-80.8 | 72.9-93.2 |

| | | | | | |
|---|---|---|---|---|---|
| *Significantly different from CC (p=0.022) | | | | | |

### Example 2: SNPs (single nucleotide polymorphisms) in the coding region of the ß, ß-carotene 15,15'-monooxygenase gene

Polymorphisms in the ß, ß-carotene 15,15'-monooxygenase gene were identified by sequencing a nucleic acid sample of patients and comparing the sequence to controls or by PCR-amplification of 400-600 base pair fragments (covering coding and regulatory regions of the ß,ß-carotene 15,15'-monooxygenase gene as defined by primer positions and PCR conditions in Table 2. Fragments were sequenced in these samples with a forward and reverse primer, polymorphisms were detected by using the PolyPhred software (licensed from University of Washington) and allele frequencies for the variants were established as described in Human Mutation (2001); 17:243-254).

**Table 2: Genomic DNA sequences of BCMO1 derived from EMZBL acc.no. AC131888.1 used for primer design of a) exons 6 and b) 8 of BCM01. Sequence in reverse complement orientation with respect to BCM01 mRNA. (Primer positions ; exons)**

| a) around exon 6 for SNP 1 (Arg267Ser) |
|---|
| 18501 |
| CCCTCTTCCA TGCCACTTCC TTGCCTTAAA CTTCACCCTC CTGCACACCA forw prim pos GCCTCCCTCC ATGGTCAGCC AGCACCCACT GGGCTAGAGT CCAGCCAGAC CTCACCTTCT CCTCCCTGTG GAAAGCCAGG CAGGAGGCCC AGCTCATTCT CCGGATGTAT GCGGTTGCCA TCTTGAGAAT ATCCAACCTG AAAGGCTGCT CAAGGAAGAT GACATAGTTC TCGGTGACTC CAAAGCTGTG GTAGTAGCTT GGGGAGAGCA GGGAGCGGGA TGGGATGGAG CAGAACACCT CTGTGTGCTT CCAGGGGCTC TTCCCCTGCT TCTTGCCCTC TAGCAAGACA AAAAGCAAGC CTCAGGAATT GTGCAGGATC TGGCTGCCCC CCCAAGACCA CACCACCATC ACCTACAAAC TGACTTGATT GCTGAAAAAA ACCTTTACAT TAAACAGCAA CGTGTAGACA GACAACAAGG TTGCTGCAGC TGCTGCAGGG GGCCAGGCTG rev prim pos AGACCTTCCA CTCAGCACCC ACCCCATATA CAATCTGAAA ACCACTGAAC |
| PCR conditions: Annealing temp. 60°C, |
| |

| b) around exon 8 for SNP2: Ala379Val |
|---|
| CCAAGACCCT TTCCTTTTTG GCCCTTGTCC ATGCACAGGA GGGAATTATT     for prim pos ATGTAAAGAG GCCTCTGGGC TCCCTGTGAA AATCTGCCCC TTTCCTAAAC TCAGGACAAG AGGATGCATT TTACCTTCAT AAAGAAATTC CGGCTGGCAG TAGACTTGGC CATCTTCTTC CTTCAGGGCC GTGGCTGTTG TAGATGCCAC TTTGATTAAA TTTGTGCCCA CTTCTGCATT CTGAAAAAAT TCAGTTTTCA AAAAGTAAAA TAAAAACGTG TAATCCAGAG CAAACATTTG CTTTAGTGTA |
| TATATTTTTT AAAATGCTTG AATACATGTC ATCTTGGCTT AGCCTCACAT TAATCATAGG AAGCAGCTCG TTTCCTGTTG TTATCCTTAG TCTCAGTTTA AAAAATGAGG ATTGGCCATT TAAGGTGACT CCTCCAAGGT CACATACCTG rev prim pos GTAGGTGATA GGACTCTTTT CTCTTACTCT GAGCCTGCCT TATCCCTCAC |
| PCR conditions: Annealing temp: 60°C |

### SNP details:

The locations of SNP 1 and SNP 2 Within the mRNA of the ß, ß-carotene 15,15'-monooxygenase gene are shown in Table 4.

SNP1 (rs12934922):
Chromosome 16q21-q23
Position: 1019 in acc.no. an:af294900.1
Allele 1: AGA - Frequency: 77% in mixed ethnicity panel
Allele 2: AGT - Frequency: 23% in mixed ethnicity panel
Amino acid position: 267
Amino acid change: Arginin -> Serin

SNP2 (rs7501331)
Chromosome 16q21-q23
Position: 1354 in acc.no. an:af 294900.1
Allele 1: GCA - Frequency: 83% in mixed ethnicity panel
Allele 2: GTA - Frequency: 17% in mixed ethnicity panel
Amino acid position: 379
Amino acid change: Alanine -> Valin

**Table 3: Complete mRNA sequence of ß, ß-carotene 15,15'-monooxygenase gene including the SNP positions for SNP1 and SNP2.**

| |
|---|
| AF294900 Length: 2446 April 14, 2004 15:23 Type: N Check: 1709 .. |
| 1 GAATGTAAAG AGATCCAGGG CTCTTGGAGA GGGACAAGTG AGAGCCAGCC 51 AAAAAGGAAA AAGCAAAGGC AGAAACGGCA TCAGGAGAGA CAGAGATGTG 101 AAGGAGGGAA GGAGCAGGAG AGCAGGAAGG AAACGCAGGA GGAGGGAGCA 151 GCATCTCCTG TGAACACAGA GGAGCACCTG TTTGCTGTTA AAATCGATCT 201 CCCTCGGCAC CCTGAGCAAT GGATATAATA TTTGGCAGGA ATAGGAAAGA 251 ACAGCTGGAG CCTGTGAGGG CCAAAGTGAC AGGCAAGATT CCAGCATGGC 301 TGCAGGGAAC CCTGCTCCGC AACGGGCCTG GGATGCACAC AGTTGGGGAG 351 TCCAGATACA ACCATTGGTT CGACGGCCTT GCCCTGCTCC ACAGCTTCAC 401 CATCAGAGAC GGTGAAGTCT ATTACAGGAG CAAATACCTG AGAAGCGATA 451 CCTACAACAC CAATATTGAG GCAAACAGGA TTGTGGTGTC TGAGTTTGGA 501 ACAATGGCCT ATCCGGACCC CTGCAAAAAC ATATTTTCCA AAGCTTTCTC 551 CTACTTGTCT CACACCATCC CCGATTTCAC CGACAACTGC CTGATCAACA 601 TCATGAAGTG CGGAGAAGAC TTCTACGCGA CCTCAGAGAC CAATTACATC 651 AGGAAAATCA ACCCACAGAC TCTGGAAACC CTGGAGAAGG TTGATTATCG 701 TAAATACGTG GCGGTAAATC TGGCAACGTC ACATCCCCAT TATGATGAGG 751 CTGGAAATGT TCTAAACATG GGCACATCCA TTGTGGAAAA GGGGAAGACA 801 AAGTATGTGA TTTTTAAGAT CCCTGCCACA GTACCAGAGG GCAAGAAGCA 851 GGGGAAGAGC CCCTGGAAGC ACACAGAGGT GTTCTGCTCC ATCCCATCCC 901 GCTCCCTGCT CTCCCCAAGC TACTACCACA GCTTTGGAGT CACCGAGAAC 951 TATGTCATCT TCCTTGAGCA GCCTTTCAGG TTGGATATTC TCAAGATGGC 1001 AACCGCATAC ATCCGGAGA(A/T) TGAGCTGGGC CTCCTGCCTG GCTTTCCACA SNP1 |
| Arg/Ser |
| 1051 GGGAGGAGAA GACTTATATC CACATCATCG ACCAAAGGAC CAGGCAGCCT 1101 GTGCAGACCA AGTTTTACAC AGACGCCATG GTGGTCTTCC ATCAGGTCAA 1151 CGCCTACGAA GAGGACGGCT GCATCGTGTT TGACGTCATT GCCTACGAGG 1201 ACAACAGCCT CTACCAGCTC TTCTACCTGG CCAACCTGAA CCAGGACTTC 1251 AAGGAGAACT CCAGGCTCAC CTCGGTCCCC ACCCTCAGGA GGTTTGCCGT 1301 GCCCCTCCAC GTGGACAAGA ATGCAGAAGT GGGCACAAAT TTAATCAAAG 1351 TGG(C/T)ATCTAC AACAGCCACG GCCCTGAAGG AAGAAGATGG CCAAGTCTAC SNP2 |
| Ala/Val |
| 1401 TGCCAGCCGG AATTTCTTTA TGAAGGCTTA GAGCTTCCAC GGGTCAATTA 1451 TGCTCACAAT GGAAAGCAAT ACCGATATGT CTTTGCTACA GGAGTTCAGT 1501 GGAGTCCAAT CCCAACCAAG ATAATAAAAT ATGACATTCT CACAAAGTCA 1551 TCCTTAAAAT GGAGAGAGGA CGACTGCTGG CCAGCGGAAC CCCTGTTTGT 1601 GCCCGCGCCA GGTGCCAAGG ATGAGGATGA CGGAGTAATC TTATCAGCCA 1651 TTGTCTCTAC TGATCCCCAA AAGCTGCCTT TTCTGCTCAT TCTGGATGCC 1701 AAAAGCTTTA CGGAATTGGC CCGTGCCTCT GTTGATGTCG ATATGCACAT 1751 GGATCTCCAT GGATTATTCA TTACAGACAT GGACTGGGAC ACAAAAAAGC 1801 AGGCCGCTTC TGAGGAACAG CGGGACAGGG CTTCCGACTG CCACGGGGCT 1851 CCTCTGACCT GATGGTGTTG GGGTTTGGGT AGGGGAGGGG AGCTCGGCTG 1901 TCAGAACTCC ATGGATATGT TTCTTTGGAT GGAGGGGAGG GCCTTTGTTA 1951 CCTTTTGCAC TTATTCTTTC TGTGGGTGCT TTGACAAGGG CATGGCAAGA 2001 GAGCTTGTCA GTATCATTTC TTTCATTTTA TTTTGGCTGT AAAAATTTTG 2051 TTTGAAAGTC AAACATTTGA ACATCAAATA TGTATTGATT AGATCCAGTC 2101 CTTCTAAGAA ACCTCCTTTC CTTTAACAAA AAGACCTTGA CCATGAATCA 2151 GAGATTGTAT TCAATGACAT TATCATCATT TTTAGAACGA GCCACTAACC 2201 TATTAGAGAA GAGGCAGATA ATTATTTATC ATTGTGCCTT CCTCTGTCTC 2251 TTTCTCTTTC CTTTGCTCCC TCCCATGTTT CTGGTGGACT AAATTGTGTA 2301 TCTGGGAGTT CTCACTTTCT AGCCAGGCCT GATGAGTTAA GTTACATGAC 2351 CAGGAAGGTT ATGCCACTTT GTGGTCATTC CATGCAAGTC ATGGCGTAAT 2401 ATAAAATAAT AAAAACTTTA AAGTCTGAAA AAAAAAAAAA AAAAA |

### Example 3: Amino acid monooxygenase including two polymorphisms

1 

51 

101 

151 

201 

251 

301 

351 

401 

451 

501 

### Example 4: Tests for enzymatic activity of different allelic forms of the human BCMO:

To express a recombinant human BCMO1 in E. coli, We cloned its CDNA by RT-PCR from a total RNA preparation derived from small intestine (Clontech, Heidelberg, Germany). Reverse transcription was carried out using 100 ng total RNA, 500 ng of an oligonucleotide T17 primer, 200 U Reverse Transcriptase (Superscriptll, Invitrogen, Karlsruhe, Germany) in a total volume of 10 µl according to the manufacturer's protocol. One µl of the reverse transcription assay was used for PCR. The PCR was carried out in a total volume of 40 µl using the oligonucleotide primers: BCO1-up: ATGGATATAATATTTGGCAGGAAT and BCO1-down: ACATATCCATGGAGTTCTGAC and one Unit Taq DNA polymerase (Amersham- Pharmacia, Freiburg, Germany). Agarose gel electrophoresis showed that a RT-PCR product of approximately 1.6 kbp was obtained. This PCR product was ligated into the expression vector pTrcHis2-TOPO using the pTrcHis2-TOPO-TA cloning kit (Invitrogen, Karlsruhe, Germany) and transformed into the E. coli strain TOP10. The vector construct was isolated and the sequence of the BCO1 cDNA insert was determined on both strands. This plasmid construct was termed pBCMO1 human. It encodes a protein of 547 amino acids. Amino acid sequence comparison revealed that it was identical to the existing BCMO sequence AAG15380 found in the data base.

To test for BCMO1 enzymatic activity, we used a bacterial test system it has been previously described (Von Lintig and Vogt, 2000). For this purpose, we transformed pBCMO1 human into an E. coli strain capable to synthesize and accumulate β,β-carotene. The bacteria were grown at 28°C in standard LB-medium until the culture reached an A600 of one. Expression of the recombinant human BCMO1 was induced by adding IPTG to a finial concentration of 1 mM for 16 h. The bacteria were harvested by centrifugation. Expression of BCMO1 was verified by Western- blot analysis using a polyclonal serum raised against the recombinant murine BCMO1. Lipids were extracted from twenty A600 bacteria and subjected to HPLC-analyses has been previously described (Von Lintig and Vogt, 2000). This analysis revealed that significant amounts of retinoids were formed at the expense of β,β-carotene upon expression of the recombinant human BCMO1. Thus, the recombinant protein was enzymatically active in the E. coli test system demonstrating that this *modus operandi* is suitable to measure human BCO1 activity in vitro.

To test for the effects of the different SNPs found in the human bcmo1 gene, we introduced these nucleotide transitions into pBCMO1 human by site directed mutatgenesis. For this we used the GeneTailor site directed mutagenesis system according to the manufacturer's protocol(Invitrogen, Karlsruhe, Germany). For site directed mutagenesis the following set of primers were used: For the A/T transition (SNP#1) which leads to an amino acid exchange from Arginine to Serine at position 267 of the deduced BCMO amino acid sequence, Ser-Mut-up GCAACCGCATACATCCGGAGTATGAGCTGGGC and Ser-Mut-down CTCCGGATGTATGCGGTTGCCATCTTGAGA. For the C/T transition (SNP#2) which leads to an amino acid exchange from Alanine to Valine in the deduced amino acid sequence of BCMO1 at position 379, Val-Mut-up CACAAATTTAATCAAAGTGGTATCTACAACAGC and Val-Mut-down CCACTTTGATTAAATTTGTGCCCACTTCTG. The nucleotide transitions in the resulting plasmids, pBCMO1 (Val379)human and pBCMO1 (Ser267)human, were verified by sequence analyses. Tests for enzymatic activity of the BCMO encoded by these two plasmids were carried out as described above. First, the expression of the recombinant BCMO1s in E. coli were verified by Western-blot analysis. HPLC analyses revealed that in both E. coli-strains expressing the mutant forms of BCMO1 the β-carotene content was elevated as compared to the E. coli-strain expressing the original BCMO1. In contrast, the retinoid content was decreased indicating that the two different SNPs influence BCO1 enzymatic activity.

## Claims

1. The use of at least one single nucleotide polymorphism (SNP) in the β, β -carotene 15,15'-dioxygenase gene for selecting a diet an individual should be provided to prevent or treat Vitamin A deficiency or Vitamin A deficiency disease (VADD) during an intervention program.

2. The use of the single nucleotide polymorphisms according to claim 1, wherein the SNP is in the coding region of the β, β -carotene 15,15'-dioxygenase gene.

3. The use according to claim 1 or 2, wherein the single nucleotide polymorphism is rs12934922 and wherein the corresponding mutation is at position 1019 resulting in an amino acid change in amino acid position No. 267 replacing an arginin with a serin.

4. The use according to any of claims 1 to 3, wherein the single nucleotide polymorphism is rs7501331 and wherein the corresponding mutation is at position 1354 resulting in an amino acid change in amino acid position No. 379 replacing an alanine with a valin.

5. The use according to claim 4, wherein the un-presence of the mutation triggers the selection of a diet an individual should be provided to prevent or treat Vitamin A deficiency or Vitamin A deficiency disease (VADD).

6. The use according to any of claims 1 to 5, wherein the individual has A deficiency or Vitamin A deficiency disease.

7. The use according to any of claims 1 to 6, wherein the diet an individual should be provided is **characterized by** an additional beta-carotene supplementation.

8. A method for the identification of an individual who has an altered risk for developing Vitamin A deficiency or VADD, the method comprising the steps of
a. providing a biological sample taken from a subject;
b. determining the nucleotides present in at least one of the polymorphic sites as set forth in claims 3 to 5 in said individual's nucleic acid; and
c. combining the SNP marker data to assess the risk of an individual to develop VADD.

9. The method according to claim 8, further comprising a step of combining the genetic information with personal and clinical information of said individual to assess the risk of an individual to develop VADD.

10. The method according to claim 9, wherein the personal and clinical information concerns age, gender, behaviour patterns and habits, biochemical measurements, clinical measurements, family history of VADD.

11. A test kit for determining the nucleotides present in at least one of the polymorphic sites as set forth in claims 3 to 5 in said individual's nucleic acid for assessment of an altered risk of VADD in a subject.
